# EUROPEAN PATENT SPECIFICATION

(11) **EP 3 157 454 B1**
(45) Date of publication and mention of the grant of the patent: **06.06.2018**
(21) Application number: 15734258.5
(22) Date of filing: 30.04.2015
(51) Int. Cl.: A61B 18/12, A61B 18/14, A61B 18/00

(54) **ELECTROSURGICAL HANDPIECE FOR RADIOFREQUENCY SURGICAL TREATMENT OF TUBO-NASAL PATHOLOGIES**
ELEKTROCHIRURGISCHES HANDSTÜCK ZUR CHIRURGISCHEN FUNKFREQUENZBEHANDLUNG VON NASENTUBUSPATHOLOGIEN
PIÈCE À MAIN ÉLECTROCHIRURGICALE POUR TRAITEMENT CHIRURGICAL PAR RADIOFRÉQUENCE DE PATHOLOGIES TUBO-NASALES

(30) Priority: 18.06.2014 IT RM20140321
(43) Date of publication of application: 26.04.2017
(73) Proprietor: Di Rienzo Businco, Lino, 00161 Roma (IT)
(72) Inventor: Di Rienzo Businco, Lino, 00161 Roma (IT)
(74) Representative: Carangelo, Pierluigi
(86) International application number: PCT/IT2015/000119
(87) International publication number: WO 2015/193923

(56) References cited:
- WO-A2-2012/014101
- JP-A- 2012 205 618
- US-A1- 2001 025 177
- US-B1- 6 451 014

## Description

The present description relates to the technical field of instruments for the electrosurgical treatment of tubo-nasal pathologies, and in particular relates to an electrosurgical handpiece for the treatment of tubo-nasal pathologies such as for example hypertrophy of the pharyngeal opening of the auricular Eustacian tube (torus tubarius) and of the middle and superior turbinates.

Reference is made to document US6451014 B1. It is increasingly common for individuals of all ages and both sexes to suffer from poor breathing associated or not with a condition of tubal stenosis with stagnation of the endotympanic mucus (catarrhal otitis). Very often the aforementioned symptoms are a direct or indirect result of tubo-nasal pathologies such as hypertrophy of the torus tubarius and/or middle turbinate and/or superior turbinate.

At present there is no minimally invasive surgical treatment available to treat hypertrophy of the torus tubarius and/or middle turbinate and/or superior turbinates. In fact, the known remedies provide for highly destructive surgery, with relative problems of bleeding and long-lasting pathological scarring. Recent epidemiological studies confirm that these diseases are on the increase in industrialised and urbanised areas, mainly because of the pollutants inhaled daily while breathing.

As known, poor breathing is often caused by a disease called, in the field of otolaryngology, turbinate hypertrophy. In particular, hypertrophy of the middle and/or superior turbinate is often the cause of sinusitis. The turbinates, of which there are three in each nasal fossa (inferior, middle and superior) are osteo-mucous structures which heat the inhaled air before it reaches the lungs, and in particular are responsible for humidifying, filtering and conditioning the inhaled air.

It is not always possible to treat turbinate hypertrophy through medications such as nasal sprays. For this reason surgical treatment techniques have been developed.

In the past, the turbinates were burned or even cut with scissors and scalpels, since their usefulness was unknown.

Recently instead highly conservative surgical treatments have been developed to reduce the volume of the inferior turbinates without cutting or sutures and especially without bleeding and thus without the annoying nasal plugs of before. To such purpose radiofrequency energy is used which is able to "deflate" the enlarged inferior turbinate without trauma and without pain, in day-surgery treatment.

These radiofrequency surgical treatments, performed with the endoscopic technique, because of their minimal invasiveness and the absence of pain, are also applicable to paediatric patients. In this age group the importance of a non-traumatic method which safeguards the functioning of the inferior turbinate without causing scars or any decrease in its valuable work of filtering the air breathed is even more evident.

The above mentioned radio frequency surgical treatments are performed using surgical systems, also known by the name of electrosurgical units, comprising a radiofrequency generator to which an electrosurgical handpiece, generally of the disposable type, is detachably associable. The aforementioned electrosurgical handpiece generally comprises a handle, for manual gripping by the surgeon and a rod with electrodes which constitutes the active part of the surgical device, i.e. the part intended to come into contact with the tissue of the turbinates during the performance of the surgical treatment.

A surgical system of the aforementioned type, as described in the patent EP 1087691 B1 has been developed by Telea Electronic Engineering and uses a technology called quantum molecular resonance (RQM). According to this technology, the cutting of the tissue is not due to the heating thereof, as is the case for the standard electro/radio scalpel, but is caused by directly breaking the molecular bonds and therefore takes place without the production of heat. As a result, the temperature of the tissue, during cutting, never exceeds 45-50°C, thus avoiding the production of necrotic tissue.

From the point of view of the radiofrequency generators, the above electrosurgical radiofrequency systems have achieved, especially with molecular quantum resonance technology, adequate and extremely satisfactory performance.

The handpieces of the known type for electrosurgical treatment of the inferior turbinates cannot unfortunately be used for treatment, by means of access through the nasal cavities, of hypertrophy of the medium and/or superior turbinates and/or torus tubarius.

The need is felt to provide a handpiece specifically designed to permit the performance of surgical treatment of hypertrophy of the medium and / or superior turbinates and / or torus tubarius in a particularly convenient and effective way with access through the nasal cavities which also provides for rotations and lateral movements of the electrodes inside the middle and / or superior turbinates and / or torus tubarius in order to better treat and reach the individual compartments to be treated even through a single hole insertion. This way it is possible to reduce the sub-mucous hypertrophy of the soft tissue underneath, without damaging the precious thin layer of mucous lining of the surface which is functionally safeguarded. Merely for the purpose of information such a technique was developed by Dr. Lino Di Rienzo Businco and is known in the sector as "Single Insertion Site".

The above-mentioned need, for which at present no suitable and / or effective handpiece exists, is fully satisfied by means of an electrosurgical handpiece as defined in general in Claim 1. Preferred and advantageous embodiments of the aforesaid electrosurgical handpiece are defined in the appended dependent claims.

The invention will be clearer to understand from the following detailed description of its particular embodiments, made by way of a non-limiting example with reference to the appended drawings, wherein:
- figure 1 is a side plane view of an electrosurgical handpiece;
- figure 2 is a partial side plane view in cross-section of an assembled part of the electrosurgical handpiece in figure 1; and
- figure 3 is a partial side plane view in cross-section of some of the parts that make up the assembled part in Figure 2.

In the appended drawings, elements which are the same or similar will be indicated using the same reference numerals.

With reference to the appended drawings, reference numeral 1 globally denotes an electrosurgical handpiece according to a possible embodiment of the present invention. According to one embodiment, the electrosurgical handpiece 1 is a disposable handpiece.

The electrosurgical handpiece 1 comprises a handle 2 which, in the example, starting from the end portion 21 has in sequence: a first substantially cylindrical portion, a second frustoconical portion having a cross-section increasing with respect to the first cylindrical portion and a third frustoconical portion with a decreasing cross-section and which extends as far as the end portion 22, so that an intermediate region 23 of the handle 2 is defined which is slightly curved and suitable to facilitate gripping between the thumb and the index and / or middle finger of a surgical operator. According to one embodiment, the aforesaid first cylindrical portion has a diameter equal to about 9.9 mm at the end portion 21 while the diameter of the handle 2 at the end portion 22 is approximately 5.5 mm.

The handle 2 is preferably made of electrically insulating material, for example in relatively rigid moulded plastic. Preferably, between the two opposite end portions 21 and 22 the handle has a length L5 of approximately 130 mm.

A cable duct sleeve 20 protrudes from the first end portion 21 of the handle 2 inside which a cable 4 is engaged to supply the handpiece 1 with a radio frequency signal. For example, such a radio frequency signal is produced by a radiofrequency generator device made according to the teachings of patent EP 1,086,691 to which the electrosurgical handpiece 1 is detachably operatively connectable via the cable 4.

The electrosurgical handpiece 1 further comprises an electrodes supporting rod 3, integral with the handle 2 and projecting from the end portion 22 thereof.

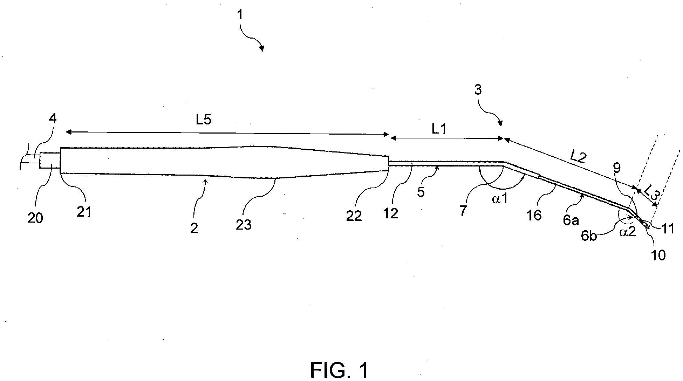

The electrodes supporting rod 3 is bent and has a first straight portion 5 adjacent to the handle 2, and a second straight portion 6a connected to the first portion 5 and bent with respect to the first portion 5, a third straight portion 6b connected to the second portion 6a bent with respect to the second portion 6a. The third portion 6b is distal from the handle 2, so that the second straight portion 6a is interposed between the first straight portion 5 and the third straight portion 6b. Preferably, as clearly shown in Figure 1, the first straight portion 5 is adjacent to the handle 2.

According to an advantageous embodiment and consistent with the example in figure 1, the third straight portion 6b has a length less than the length of the first straight portion 5 and that of the second straight portion 6a.

According to one embodiment, the first portion 5 of the electrodes supporting rod 3 has an overall length L1 comprised between 40 mm and 50 mm inclusive and preferably equal to 45 mm or equal to about 45 mm.

According to one embodiment, the second portion 6a of the electrodes supporting rod 3 has an overall length L2 comprised between 47 mm and 67 mm inclusive and preferably equal to 57 mm or equal to about 57 mm.

According to one embodiment, the third portion 6b of the rod 3 has an overall length L3 comprised between 5 mm and 15 mm inclusive and preferably equal to 10 mm or equal to about 10 mm.

The first portion 5 of the electrodes supporting rod 3 is preferably coaxial to the handle 2.

Preferably, the second portion 6a of the electrodes supporting rod 3 is bent with respect to the first portion 5 according to an inner bending angle α1 comprised between 140° and 160°, inclusive, and preferably equal to 150° or approximately 150°. The third portion 6b of the electrodes supporting rod 3 is bent with respect to the second section 6a according to an inner bending angle α2 comprised between 130° and 170° inclusive and preferably equal to 150° or approximately 150°.

Preferably the first portion 5, the second portion 6a and the third portion 6b lie on the same plane. More preferably, in the above embodiment, as shown in the example represented in figure 1, the third portion 6b is bent with respect to the second portion 6a in the same direction in which the second portion 6a is bent with respect to the first portion 5.

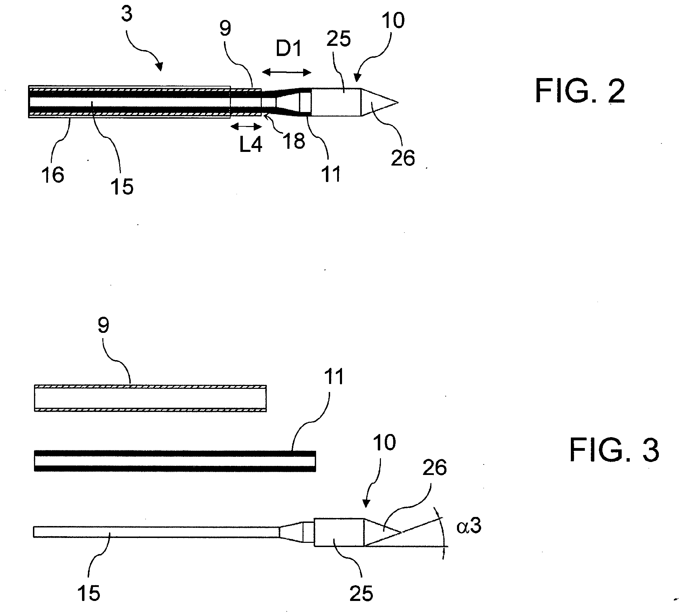

The third portion 6b has a free end portion comprising two electrodes 9 and 10 separated by a first sheath of insulating coating 11. With reference to figure 2, according to one embodiment, the minimum distance D1 between the exposed part of the electrodes 9 and 10 is equal to 2 mm.

According to one embodiment, with reference to figures 1 and 2, the electrodes 9 and 10 are coaxial to the third straight portion 6b.

With reference to figures 2 and 3, according to a preferred embodiment the electrodes supporting rod 3 comprises the following elements arranged coaxially with each other:
- a central conductor 15, for example in stainless steel the free end of which, enlarged with respect to a remaining portion of the central conductor 15, constitutes the electrode 10;
- the first covering sheath 11 of insulating material, for example polyamide, adhering to the central conductor 15 and such as to leave exposed the electrode 10, being externally substantially flush with said electrode (figure 2);
- a tubular conductor 9 placed immediately outside the first covering sheath 11, the exposed portion (portion L4 in figure 2)of which forms the second electrode 9;
- a second covering sheath 16 of insulating material adhering to the outside of the tubular conductor 9, preferably transparent;
- an outer tubular reinforcing element 12 (visible in figure 1) placed outside the second covering sheath 16 and suitable to entirely cover the first portion 5 of the electrodes supporting rod 3 and to cover partially the second portion 6a (preferably by an amount equal to about 15 mm).

According to a particularly advantageous embodiment, the second covering sheath 16 is such as to leave exposed the tubular conductor 9 so that the exposed part of the electrode 9 has a length of between 1 mm and 4 mm and preferably equal to about 2 mm. This advantageously makes it possible to prevent that during surgery tissue portions different from those specified which need or are planned to be treated, are subjected to radiofrequency treatment.

Preferably, the outer tubular reinforcement element 12 is made of stainless steel.

Preferably, all the above listed elements of the electrodes supporting rod 3 arranged coaxially to each other each have an end portion placed inside the handle 2 being embedded therein.

With reference to figure 2, according to one embodiment, the exposed part of the two electrodes 9 and 10 and the exposed part of the first covering sheath 11 are externally substantially flush with each other. This clearly except in the case of a possible step 18 of negligible height which would tend in any case to be generally offset by the elastic expansion of the covering sheath 11, if this is for example made of relatively elastic material such as for example polyamide.

With reference to figure 3, according to a further advantageous embodiment the electrode 10 has a cylindrical part 25, preferably having a length of about 2 mm, and a tapered tip 26, preferably having a length equal to about 1.5 mm. Preferably, the tapered tip 26 has a taper angle of approximately 140° so that the outer angle α3 represented in figure 3 is equal to about 20°.

Experimental tests have made it possible to verify that an electrosurgical handpiece as described above makes it possible to perform with particular efficiency, comfort and precision electrosurgical treatment for hypertrophy both of the middle turbinates, the superior turbinates and the torus tubarius, with access from the nasal cavity, in particular according to intervention techniques that provide for the rotations of the electrodes or of the lateral movements thereof in order to selectively reach the various compartments to be treated, particularly but not exclusively, if used in combination with a molecular quantum resonance radiofrequency generator and preferably also with the aid of a probe suitable to allow a surgeon to view the area to be treated.

Obviously, a person skilled in the art may make further modifications and variations to the electrosurgical handpiece described above so as to satisfy contingent and specific requirements while remaining within the sphere of protection of the invention as defined by the following claims.

## Claims

1. Electrosurgical handpiece (1) for the radiofrequency surgical treatment of tubo-nasal pathologies, such as hypertrophy of the middle and/or superior nasal turbinates and/or of the torus tubarius, comprising a handle (2) and an electrodes supporting rod (3) firmly attached to the handle (2) and projecting from an end portion (22) thereof, the electrodes supporting rod (3) being bent and comprising:
- a first straight portion (5) adjacent to the handle (2) ;
- a second straight portion (6a) connected to the first portion (5) and bent in relation to the first portion (5);
- a third straight portion (6b) connected to the second portion (6a), bent with respect to the second portion (6a) and comprising a free end portion fitted with two electrodes (9, 10), wherein:
- the first straight portion (5), the second straight portion (6a) and the third straight portion (6b) lie on the same plane;
- the third straight portion (6b) is bent with respect to the second straight portion (6a) in the same direction in which the second straight portion (6a) is bent with respect to the first straight portion (5);
- the third straight portion (6b) has a length less than the length of the first straight portion (5) and that of the second straight portion (6a);
- the first straight portion (5) is adjacent to the handle;
- the second straight portion (6a) is interposed between the first straight portion (5) and the third straight portion (6b).

2. Electrosurgical handpiece (1) according to claim 1, wherein the second portion (6a) has a length (L2) comprised between 47 mm and 67 mm inclusive.

3. Electrosurgical handpiece (1) according to claims 1 or 2, wherein the third portion (6b) has a length (L3) comprised between 5 mm and 10 mm inclusive.

4. Electrosurgical handpiece (1) according to any of the previous claims, wherein said first portion (5) and said second portion (6a) are bent with each other according to an inner bending angle (α1) comprised between 140° and 160° inclusive.

5. Electrosurgical handpiece (1) according to any of the previous claims, wherein said second portion (6a) and said third portion (6b) are bent with each other according to an inner bending angle (α2) comprised between 130° and 170° inclusive.

6. Electrosurgical handpiece (1) according to any of the previous claims, wherein said electrodes (9, 10) are separated from each other by a first sheath of insulating coating (11) and wherein the minimum distance between an exposed part of said electrodes (9, 10) is equal to 2 mm.

7. Electrosurgical handpiece (1) according to any of the previous claims, wherein one of said electrodes (10) has a cylindrical part (25) and a conical tip (26).

8. Electrosurgical handpiece (1) according to any of the previous claims, wherein the second covering sheath (16) is such as to leave exposed the tubular conductor (9) so that the second electrode (9) has an exposed part of a length comprised between 1 mm and 4 mm.

9. Electrosurgical handpiece (1) according to any of the previous claims, wherein said electrodes (9,10) are coaxial with respect to the third straight portion (6b).

10. Electrosurgical system, comprising a radiofrequency generator and an electrosurgical handpiece (1) according to any of the previous claims detachably connectable to said generator.

11. Electrosurgical system according to claim 10, wherein said generator is a quantum molecular resonance generator.

## Patentansprüche

1. Elektrochirurgisches Handgerät (1) zur hochfrequenzchirurgischen Behandlung von nasal-tubalen Pathologien, wie beispielsweise Hypotrophie der mittleren und/oder oberen Nasenmuscheln und/oder des Torus Tubarius, aufweisend einen Griff (2) und einen fest an dem Griff (2) befestigten und von einem Endbereich (22) des Griffs (2) vorragenden Elektrodenhaltestab (3), wobei der Elektrodenhaltestab (3) gebogen ist und aufweist:
- einen ersten geraden Bereich (5) neben dem Griff (2);
- einen mit dem ersten Bereich (5) verbundenen und bezüglich dem ersten Bereich (5) abgewinkelten zweiten geraden Bereich (6a);
- einen mit dem zweiten Bereich (6a) verbundenen, bezüglich dem zweiten Bereich (6a) abgewinkelten und einen mit zwei Elektroden (9, 10) versehenen freien Endbereich aufweisenden dritten geraden Bereich (6b),
wobei:
- der erste gerade Bereich (5), der zweite gerade Bereich (6a) und der dritte gerade Bereich (6b) in derselben Ebene liegen;
- der dritte gerade Bereich (6b) bezüglich dem zweiten geraden Bereich (6a) in derselben Richtung abgewinkelt ist, in der der zweite gerade Bereich (6a) bezüglich dem ersten geraden Bereich (5) abgewinkelt ist;
- der dritte gerade Bereich (6b) eine Länge aufweist, die geringer ist als die Länge des ersten geraden Bereichs (5) und die des zweiten geraden Bereichs (6a);
- der erste gerade Bereich (5) neben dem Griff angeordnet ist;
- der zweite gerade Bereich (6a) zwischen dem ersten geraden Bereich (5) und dem dritten geraden Bereich (6b) angeordnet ist.

2. Elektrochirurgisches Handgerät (1) gemäß Anspruch 1, wobei der zweite Bereich (6a) eine Länge (L2) zwischen einschließlich 47 mm und 67 mm aufweist.

3. Elektrochirurgisches Handgerät (1) gemäß Anspruch 1 oder 2, wobei der dritte Bereich (6b) eine Länge (L3) zwischen einschließlich 5 mm und 10 mm aufweist.

4. Elektrochirurgisches Handgerät (1) gemäß einem der vorangehenden Ansprüche, wobei der erste Bereich (5) und der zweite Bereich (6a) in Bezug zueinander in einem inneren Biegungswinkel (α1) zwischen einschließlich 140° und 160° abgewinkelt sind.

5. Elektrochirurgisches Handgerät (1) gemäß einem der vorangehenden Ansprüche, wobei der zweite Bereich (6a) und der dritte Bereich (6b) in Bezug zueinander in einem inneren Biegungswinkel (α2) zwischen einschließlich 130° und 170° abgewinkelt sind.

6. Elektrochirurgisches Handgerät (1) gemäß einem der vorangehenden Ansprüche, wobei die Elektroden (9, 10) durch eine erste Abschirmung aus einer isolierenden Beschichtung (11) voneinander getrennt sind und wobei der minimale Abstand zwischen einem freiliegenden Bereich der Elektroden (9, 10) gleich 2 mm ist.

7. Elektrochirurgisches Handgerät (1) gemäß einem der vorangehenden Ansprüche, wobei eine der Elektroden (10) ein zylindrisches Teil (25) und eine konische Spitze (26) aufweist.

8. Elektrochirurgisches Handgerät (1) gemäß einem der vorangehenden Ansprüche, wobei die zweite Abschirmabdeckung (16) derart ausgestaltet ist, dass sie den röhrenförmigen Leiter (9) freiliegend lässt, so dass die zweite Elektrode (9) einen freiliegenden Bereich mit einer Länge zwischen 1 mm und 4 mm aufweist.

9. Elektrochirurgisches Handgerät (1) gemäß einem der vorangehenden Ansprüche, wobei die Elektroden (9, 10) zu dem dritten geraden Bereich (6b) koaxial sind.

10. Elektrochirurgisches System aufweisend einen Hochfrequenzgenerator und ein mit dem Generator trennbar verbindbares elektrochirurgisches Handgerät (1) nach einem der vorangehenden Ansprüche.

11. Elektrochirurgisches System gemäß Anspruch 10, wobei der Generator ein molekularer Quantenresonanzgenerator ist.

## Revendications

1. Pièce à main électro-chirurgicale (1) pour le traitement chirurgical par radiofréquence de pathologies tubo-nasales, telles qu'une hypertrophie des cornets moyen et/ou supérieur et/ou du bourrelet tubaire, comprenant une poignée (2) et une tige de support d'électrodes (3) montée fermement à la poignée (2) et faisant saillie à partir d'une partie d'extrémité (22) de celle-ci, la tige de support d'électrodes (3) étant coudée et comprenant :
- une première partie rectiligne (5) adjacente à la poignée (2) ;
- une deuxième partie rectiligne (6a) reliée à la première partie (5) et coudée par rapport à la première partie (5) ;
- une troisième partie rectiligne (6b) reliée à la deuxième partie (6a) coudée par rapport à la deuxième partie (6a) et comprenant une partie d'extrémité libre équipée de deux électrodes (9, 10),
dans laquelle :
- la première partie rectiligne (5), la deuxième partie rectiligne (6a) et la troisième partie rectiligne (6b) reposent sur le même plan ;
- la troisième partie rectiligne (6b) est coudée par rapport à la deuxième partie rectiligne (6a) dans la même direction dans laquelle la deuxième partie rectiligne (6a) est coudée par rapport à la première partie rectiligne (5) ;
- la troisième partie rectiligne (6b) a une longueur inférieure à la longueur de la première partie rectiligne (5) et à celle de la deuxième partie rectiligne (6a) ;
- la première partie rectiligne (5) est adjacente à la poignée ;
- la deuxième partie rectiligne (6a) est interposée entre la première partie rectiligne (5) et la troisième partie rectiligne (6b).

2. Pièce à main électro-chirurgicale (1) selon la revendication 1, dans laquelle la deuxième partie (6a) a une longueur (L2) allant de 47 mm à 67 mm inclus.

3. Pièce à main électro-chirurgicale (1) selon les revendications 1 ou 2, dans laquelle la troisième partie (6b) a une longueur (L3) allant de 5 mm à 10 mm inclus.

4. Pièce à main électro-chirurgicale (1) selon l'une quelconque des revendications précédentes, dans laquelle ladite première partie (5) et ladite deuxième partie (6a) sont coudées l'une part rapport à l'autre suivant un angle de courbure interne (α1) allant de 140° à 160° inclus.

5. Pièce à main électro-chirurgicale (1) selon l'une quelconque des revendications précédentes, dans laquelle ladite deuxième partie (6a) et ladite troisième partie (6b) sont coudées l'une par rapport à l'autre suivant un angle de courbure interne (α2) allant de 130° à 170° inclus.

6. Pièce à main électro-chirurgicale (1) selon l'une quelconque des revendications précédentes, dans laquelle lesdites électrodes (9, 10) sont séparées l'une de l'autre par une première gaine de revêtement isolant (11) et dans laquelle la distance minimale entre une partie exposée desdites électrodes (9, 10) est égale à 2 mm.

7. Pièce à main électro-chirurgicale (1) selon l'une quelconque des revendications précédentes, dans laquelle l'une desdites électrodes (10) a une partie cylindrique (25) et une pointe conique (26).

8. Pièce à main électro-chirurgicale (1) selon l'une quelconque des revendications précédentes, dans laquelle la seconde gaine de couverture (16) est conçue de sorte à laisser exposé le conducteur tubulaire (9) de sorte que la seconde électrode (9) présente une partie exposée d'une longueur allant de 1 mm à 4 mm.

9. Pièce à main électro-chirurgicale (1) selon l'une quelconque des revendications précédentes, dans laquelle lesdites électrodes (9, 10) sont coaxiales par rapport à la troisième partie rectiligne (6b).

10. Système électro-chirurgical, comprenant un générateur de radiofréquence et une pièce à main électro-chirurgicale (1) selon l'une quelconque des revendications précédentes, apte à être montée de manière amovible sur ledit générateur.

11. Système électro-chirurgical selon la revendication 10, dans lequel ledit générateur est un générateur quantique moléculaire à résonance.
